# EUROPEAN PATENT APPLICATION

(11) **EP 3 838 006 A1**
(43) Date of publication of application: **23.06.2021**
(21) Application number: 19218507.2
(22) Date of filing: 20.12.2019
(51) Int. Cl.: A24F 40/10, A24F 40/65, A24F 40/60, A24F 40/50, A61M 11/04, H04M 1/725

(54) **A SMOKING SUBSTITUTE DEVICE AND METHOD FOR MANAGING A SMOKING SUBSTITUTE DEVICE**

(71) Applicant: NERUDIA LIMITED, Liverpool Merseyside L24 9HP (GB)
(72) Inventor: The designation of the inventor has not yet been filed
(74) Representative: Mewburn Ellis LLP

(57) **Abstract**

A smoking substitute device is configured to be wirelessly triggered to issue an alert to enable a user to locate the device. The alert can be deactivated automatically by moving the smoking substitute device in a predetermined movement. Advantageously, the user does not have to input a user command into a remote device from which an alert command is sent in order to deactivate the alert. The smoking substitute device includes an alert device that is triggered to issue an alert between a first event and a second event. Suitably, the first event trigger is the receiving of an alert command by a wireless interface of the smoking substitute device. The smoking substitute device includes a motion sensor, for instance an accelerometer for detecting motion of the smoking substitute device. Advantageously, the second event trigger is the detection by the motion sensor that the smoking substitute device has been moved in a predetermined movement.

## Description

### TECHNICAL FIELD

The present invention relates to a smoking substitute device and particularly, although not exclusively, to the management of the smoking substitute device to assist a user in locating the device.

### BACKGROUND

The smoking of tobacco is generally considered to expose a smoker to potentially harmful substances. It is generally thought that a significant amount of the potentially harmful substances are generated through the heat caused by the burning and/or combustion of the tobacco and the constituents of the burnt tobacco in the tobacco smoke itself.

Conventional combustible smoking articles, such as cigarettes, typically comprise a cylindrical rod of tobacco comprising shreds of tobacco which is surrounded by a wrapper, and usually also a cylindrical filter axially aligned in an abutting relationship with the wrapped tobacco rod. The filter typically comprises a filtration material which is circumscribed by a plug wrap. The wrapped tobacco rod and the filter are joined together by a wrapped band of tipping paper that circumscribes the entire length of the filter and an adjacent portion of the wrapped tobacco rod. A conventional cigarette of this type is used by lighting the end opposite to the filter, and burning the tobacco rod. The smoker receives mainstream smoke into their mouth by drawing on the mouth end or filter end of the cigarette.

Combustion of organic material such as tobacco is known to produce tar and other potentially harmful byproducts. There have been proposed various smoking substitute devices in order to avoid the smoking of tobacco.

Such smoking substitute devices can form part of nicotine replacement therapies aimed at people who wish to stop smoking and overcome a dependence on nicotine.

Smoking substitute devices may comprise electronic systems that permit a user to simulate the act of smoking by producing an aerosol (also referred to as a "vapour") that is drawn into the lungs through the mouth (inhaled) and then exhaled. The inhaled aerosol typically bears nicotine and/or flavourings without, or with fewer of, the odour and health risks associated with traditional smoking.

In general, smoking substitute devices are intended to provide a substitute for the rituals of smoking, whilst providing the user with a similar experience and satisfaction to those experienced with traditional smoking and tobacco products. Some smoking substitute systems use smoking substitute articles (also referred to as a "consumables") that are designed to resemble a traditional cigarette and are cylindrical in form with a mouthpiece at one end.

The popularity and use of smoking substitute devices has grown rapidly in the past few years. Although originally marketed as an aid to assist habitual smokers wishing to quit tobacco smoking, consumers are increasingly viewing smoking substitute devices as desirable lifestyle accessories. Some smoking substitute devices are designed to resemble a traditional cigarette and are cylindrical in form with a mouthpiece at one end. Other smoking substitute devices do not generally resemble a cigarette (for example, the smoking substitute device may have a generally box-like form).

There are a number of different categories of smoking substitute devices, each utilising a different smoking substitute approach. A smoking substitute approach corresponds to the manner in which the substitute system operates for a user.

One approach for a smoking substitute device is the so-called "vaping" approach, in which a vapourisable liquid, typically referred to (and referred to herein) as "e-liquid", is heated by a heating device to produce an aerosol vapour which is inhaled by a user. An e-liquid typically includes a base liquid as well as nicotine and/or flavourings. The resulting vapour therefore typically contains nicotine and/or flavourings. The base liquid may include propylene glycol and/or vegetable glycerin.

A typical vaping smoking substitute device includes a mouthpiece, a power source (typically a battery), a tank for containing e-liquid, as well as a heating device. In use, electrical energy is supplied from the power source to the heating device, which heats the e-liquid to produce an aerosol (or "vapour") which is inhaled by a user through the mouthpiece.

Vaping smoking substitute devices can be configured in a variety of ways. For example, there are "closed system" vaping smoking substitute devices which typically have a sealed tank and heating element which is pre-filled with e-liquid and is not intended to be refilled by an end user. One subset of closed system vaping smoking substitute devices include a main body which includes the power source, wherein the main body is configured to be physically and electrically coupled to a consumable including the tank and the heating element. In this way, when the tank of a consumable has been emptied, the main body can be reused by connecting it to a new consumable. Another subset of closed system vaping smoking substitute devices are completely disposable, and intended for one-use only.

There are also "open system" vaping smoking substitute devices which typically have a tank that is configured to be refilled by a user, so the device can be used multiple times.

An example vaping smoking substitute device is the myblu™ e-cigarette. The myblu™ e-cigarette is a closed system device which includes a main body and a consumable. The main body and consumable are physically and electrically coupled together by pushing the consumable into the main body. The main body includes a rechargeable battery. The consumable includes a mouthpiece, a sealed tank which contains e-liquid, as well as a heating device, which for this device is a heating filament coiled around a portion of a wick which is partially immersed in the e-liquid. The device is activated when a microprocessor on board the main body detects a user inhaling through the mouthpiece. When the device is activated, electrical energy is supplied from the power source to the heating device, which heats e-liquid from the tank to produce a vapour which is inhaled by a user through the mouthpiece.

Another example vaping smoking substitute device is the blu PRO™ e-cigarette. The blu PRO™ e-cigarette is an open system device which includes a main body, a (refillable) tank, and a mouthpiece. The main body and tank are physically and electrically coupled together by screwing one to the other. The mouthpiece and refillable tank are physically coupled together by screwing one of the other, and detaching the mouthpiece from the refillable tank allows the tank to be refilled with e-liquid. The device is activated by a button on the main body. When the device is activated, electrical energy is supplied from the power source to a heating device, which heats e-liquid from the tank to produce a vapour which is inhaled by a user through the mouthpiece.

Another approach for a smoking substitute system is the so-called Heated Tobacco ("HT") approach in which tobacco (rather than an "e-liquid") is heated or warmed to release vapour. HT is also known as "heat not burn" ("HNB"). The tobacco may be leaf tobacco or reconstituted tobacco. The vapour may contain nicotine and/or flavourings. In the HT approach the intention is that the tobacco is heated but not burned, i.e. the tobacco does not undergo combustion.

A typical HT smoking substitute system may include a device and a consumable. The consumable may include the tobacco material. The device and consumable may be configured to be physically coupled together. In use, heat may be imparted to the tobacco material by a heating element of the device, wherein airflow through the tobacco material causes components in the tobacco material to be released as vapour. A vapour may also be formed from a carrier in the tobacco material (this carrier may for example include propylene glycol and/or vegetable glycerine) and additionally volatile compounds released from the tobacco. The released vapour may be entrained in the airflow drawn through the tobacco.

As the vapour passes through the consumable (entrained in the airflow) from the location of vaporisation to an outlet of the consumable (e.g. a mouthpiece), the vapour cools and condenses to form an aerosol for inhalation by the user. The aerosol will normally contain the volatile compounds.

In HT smoking substitute systems, heating as opposed to burning the tobacco material is believed to cause fewer, or smaller quantities, of the more harmful compounds ordinarily produced during smoking. Consequently, the HT approach may reduce the odour and/or health risks that can arise through the burning, combustion and pyrolytic degradation of tobacco.

There may be a need for improved design of smoking substitute systems, in particular HT smoking substitute systems, to enhance the user experience and improve the function of the HT smoking substitute system.

An example of the HT approach is the IQOS™ smoking substitute device from Philip Morris Ltd. The IQOS™ smoking substitute device uses a consumable, including reconstituted tobacco located in a wrapper. The consumable includes a holder incorporating a mouthpiece. The consumable may be inserted into a main body that includes a heating device. The heating device has a thermally conductive heating knife which penetrates the reconstituted tobacco of the consumable, when the consumable is inserted into the heating device. Activation of the heating device heats the heating element (in this case a heating knife), which, in turn, heats the tobacco in the consumable. The heating of the tobacco causes it to release nicotine vapour and flavourings which may be drawn through the mouthpiece by the user through inhalation.

A second example of the HT approach is the device known as "Glo"™ from British American Tobacco p.l.c. Glo™ comprises a relatively thin consumable. The consumable includes leaf tobacco which is heated by a heating device located in a main body. When the consumable is placed in the main body, the tobacco is surrounded by a heating element of the heating device. Activation of the heating device heats the heating element, which, in turn, heats the tobacco in the consumable. The heating of the tobacco causes it to release nicotine vapour and flavourings which may be drawn through the consumable by the user through inhalation. The tobacco, when heated by the heating device, is configured to produce vapour when heated rather than when burned (as in a smoking apparatus, e.g. a cigarette). The tobacco may contain high levels of aerosol formers (carrier), such as vegetable glycerine ("VG") or propylene glycol ("PG").

The present inventor(s) have observed that most smoking substitute devices currently on the market are configured to operate in isolation of other devices, which limits the functions the smoking substitute devices can perform.

If the smoking substitute device does not operate in isolation to other devices, it may be possible to link the other device to the smoking substitute device by a wireless communication and use the other device to trigger an alert signal to assist a user in locating the smoking substitute device. Here, it would be advantageous to provide an effective user convenience when deactivating the alert signal.

The present invention has been devised in light of the above considerations.

### Summary of the Invention

According to exemplary aspects, a smoking substitute device is configured to be wirelessly triggered to issue an alert to enable a user to locate the device. The alert can be deactivated automatically by moving the smoking substitute device in a predetermined movement. Advantageously, the user does not have to input a user command into a remote device from which an alert command is sent in order to deactivate the alert.

At their most general, the exemplary aspects provide a smoking substitute device, a system including the smoking substitute device and the method of managing the smoking substitute device wherein the smoking substitute device includes an alert device that is triggered to issue an alert between a first event trigger and a second event trigger. Suitably, the first event trigger is the receiving of an alert command by a wireless interface of the smoking substitute device. The smoking substitute device includes a motion sensor, for instance an accelerometer for detecting motion of the smoking substitute device. Advantageously, the second event trigger is the detection by the motion sensor that the smoking substitute device has been moved in a predetermined movement.

There is therefore provided, according to a first exemplary embodiment, a smoking substitute device having a wireless communication interface, an alert device, a motion sensor, and a control unit. Suitably, according to exemplary embodiments, the wireless communication interface, alert device, motion sensor and control unit are housed in a body.

The wireless communication interface is configured to wirelessly communicate with an external device. Specifically, the wireless communication interface is arranged to establish a wireless communication link with the external device. Here, the wireless communication interface is configured to receive an alert command. That is, the wireless communication interface is configured to receive an alert command from the external device. Here, the wireless communication interface receives the alert command over the established wireless communication link. Typically, receiving the alert command is completed under the control of the control unit. Suitably, the external device may be a smartphone, tablet, smartwatch or other suitable device. The control unit may complete a pairing operation to pair the smoking substitute device and external device or to otherwise verify the external device as a trusted external device that is able to transmit commands to the smoking substitute device. In exemplary embodiments, the wireless communication interface and wireless communication link are short distance communication links such as a wireless personal area network.

In the exemplary embodiments, the smoking substitute device comprises an alert device. The alert device is arranged so that when activated, the alert device issues an alert. Suitably, the alert is an audible alert. Additionally or alternatively, the alert is a visual alert. Thus, the alert is able to assist guide a user to the device's location by providing the user with a visual and / or audible location identifier.

In the exemplary embodiments including an audible alert, the alert device may suitably comprise a speaker. Here, the speaker may be arranged to emit a sound periodically between the first and second event triggers. For instance the emission of a repeated beep or buzzer or other audible noise to a user. Or the speaker may be arranged to make a substantially continuous noise between the first and second events. For instance, to play music. In the exemplary embodiments, the audible sound is issued as an alert to guide the user to the smoking substitute device.

In the exemplary embodiments including an audible alert, the alert device may additionally or alternatively include a haptic generator. Here, the haptic generator creates a vibratory force that causes the smoking substitute device to vibrate. The vibrating device typically causes an audible sound to emanate. Thus an audible signal to the device's location is issued. In addition to the audible alert, the haptic generator also creates a haptic alert that a user may sense. For instance by sensing the vibrations should the smoking substitute device be in connected contact, for instance by being in a user's pocket or the like. Suitably, the haptic generator is a vibrating element such as a haptic motor. In some embodiments, the haptic motor is an electric motor and a weight mounted eccentrically on a shaft of the electric motor. The haptic generator may be arranged to emit a constant vibration or a periodic vibration between the first and second events.

In the exemplary embodiments including a visual alert, the alert device suitably comprises a light emitter. For instance the light emitter may be a light emitting device. Suitably, the light emitter may be controllable to emit light of varying wavelengths. Thus the light emitted can be controlled to change colour. In the exemplary embodiments, the alert device is configured to emit a visual alert, for instance a light between the first event and the second event. The emitted light may be a constant light emission or a periodic flash.

In the exemplary embodiments, the smoking substitute device comprises a motion sensor. The motion sensor is arranged to detect motion of the smoking substitute device. For instance, the motion sensor is suitably an accelerometer. The motion sensor is arranged to produce signals in response to a movement of the smoking substitute device. Suitably, the control unit monitors the response signals from the motion sensor and determines a movement of the smoking substitute device by analysing the signals. Here, the control unit can determine when predetermined movement of the smoking substitute device is replicated. In doing so, the control unit can determine the second event as the occurrence of a predetermined movement of the smoking substitute device. Suitably, the second event is configured to be a movement of lifting or picking up the smoking substitute device. Here, the movement may be determined to be a specific distance of movement. For instance, by monitoring the movement time and / or movement velocity and / or movement direction. In exemplary embodiments, the control unit determines a predetermined movement has occurred when predetermined values are met or exceeded.

The control unit controls the operation of the smoking substitute device. Thus the control unit controls the receipt of the alert command by the wireless communication interface, and the activation and deactivation of the alert device upon recognition of the first and second event triggers respectively. In one exemplary embodiment, the control unit is configured to deactivate the alert device on the second event trigger and to also control the wireless interface to issue a found command to the external device.

According to a further exemplary embodiment, there is provided a system for managing the smoking substitute device comprising an external device and the smoking substitute device. The external device suitably operates an application to control the communication with the smoking substitute device and to display the information to the user. The application suitably provides an input means through the application's graphic user interface to initiate the sending of an alert command to the connected smoking substitute device. In one exemplary embodiment, the application is configured to verify the smoking substitute device is connected to the external device by a short distance wireless communication prior to sending the alert command. If the short distance wireless communication link is not established an error message is suitably provided to the user.

According to a further exemplary embodiment, there is provided a method of managing a smoking substitute device. The method comprises the steps of activating an alert device to issue an alert upon identifying a first event trigger and deactivating the alert device to stop issuing the alert upon identifying a second event trigger, wherein the second event trigger is a motion sensor detecting a predetermined movement of the smoking substitute device. In the exemplary embodiments, the method comprises receiving an alert command. Here the alert command is received by the wireless interface of the smoking substitute device and the control unit identifies the first event trigger as the receipt of the alert command. In exemplary embodiments, the method comprises causing the alert device to issue an audible alert and / or a visual alert and / or a haptic alert.

According to exemplary embodiments of the management method, the method comprises the step of the user inputting an alert input into the external device. Suitably, the external device verifies a short distance wireless communication link is established between the external device and the smoking substitute device and if a link is established the alert command is issued or if a link is not established the external device issues an error message to the user.

According to another exemplary aspect, there is provided a computer implemented method for managing a smoking substitute device to execute the previous method aspect or a computer-readable medium containing computer-readable instructions which, when executed by a processor, cause the processor to perform the precious method aspect.

The invention includes the combination of the aspects and preferred features described except where such a combination is clearly impermissible or expressly avoided.

The skilled person will appreciate that except where mutually exclusive, a feature or parameter described in relation to any one of the above aspects may be applied to any other aspect. Furthermore, except where mutually exclusive, any feature or parameter described herein may be applied to any aspect and/or combined with any other feature or parameter described herein.

### Summary of the Figures

Embodiments and experiments illustrating the principles of the invention will now be discussed with reference to the accompanying figures in which:
**Figure 1** shows an example system for managing a smoking substitute device;
**Figure 2(a)** shows an example smoking substitute device for use as the smoking substitute device in the system of Fig. 1;
**Figure 2(b)** shows the main body of the smoking substitute device of Fig. 2(a) without the consumable;
**Figure 2(c)** shows the consumable of the smoking substitute device of Fig. 2(a) without the main body;
**Figure 3(a)** is a schematic view of the main body of the smoking substitute device of Fig. 2(a);
**Figure 3(b)** is a schematic view of the consumable of the smoking substitute device of Fig. 2(a);
**Figure 4** is an example schematic view of a system for managing the automatic alert deactivation of a smoking substitute device according to an exemplary embodiment; and
**Figure 5** is a flow chart of an automatic alert deactivation method according to an exemplary embodiment.

### Detailed Description of the Invention

Aspects and embodiments of the present invention will now be discussed with reference to the accompanying figures. Further aspects and embodiments will be apparent to those skilled in the art. All documents mentioned in this text are incorporated herein by reference.

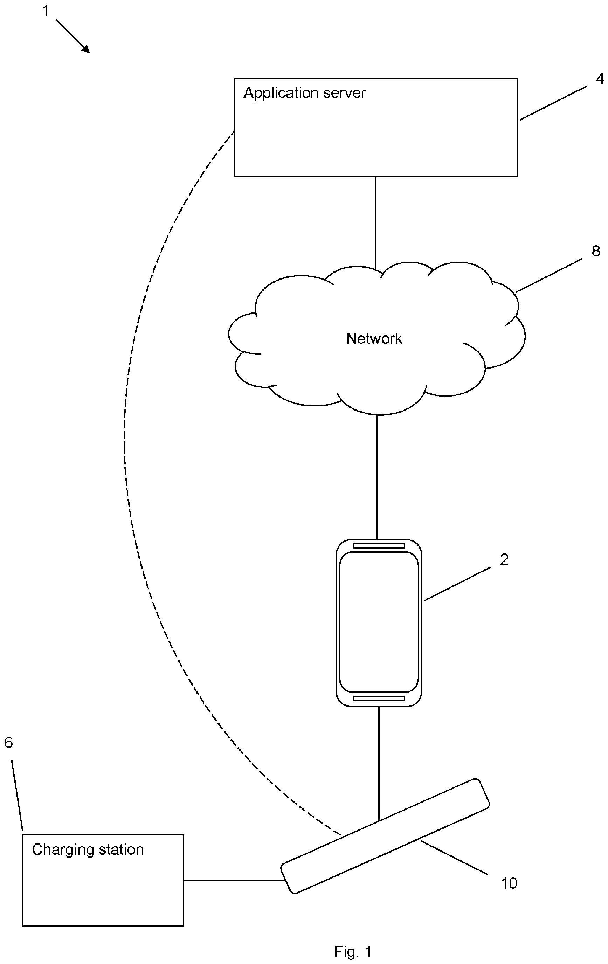

Fig. 1 shows an example system 1 for managing a smoking substitute device 10.

The system 1 as shown in Fig. 1 includes a mobile device 2, an application server 4, an optional charging station 6, as well as the smoking substitute device 10.

The smoking substitute device 10 is configured to communicate wirelessly, e.g. via Bluetooth™, with an application (or "app") installed on the mobile device 2, e.g. via a suitable wireless interface (not shown) on the mobile device 2. The mobile device 2 may be a mobile phone, for example. The application on the mobile phone is configured to communicate with the application server 4, via a network 8. The application server 4 may utilise cloud storage, for example.

The network 8 may include a cellular network and/or the internet.

In other examples, the smoking substitute device 10 may be configured to communicate with the application server 4 via a connection that does not involve the mobile device 2, e.g. via a narrowband internet of things ("NB-loT") connection. In some examples, the mobile device 2 may be omitted from the system.

A skilled person would readily appreciate that the mobile device 2 may be configured to communicate via the network 8 according to various communication channels, preferably a wireless communication channel such as via a cellular network (e.g. according to a standard protocol, such as 3G or 4G) or via a WiFi network.

The app installed on the mobile device and the application server 4 may be configured to assist a user with their smoking substitute device 10, based on information communicated between the smoking substitute device 10 and the app and/or information communicated between the app and the application server 4. The app may run in the background to handle communication with the smoking substitute device.

The charging station 6 (if present) may be configured to charge (and optionally communicate with) the smoking substitute device 10, via a charging port on the smoking substitute device 10. The charging port on the smoking substitute device 10 may be a USB port, for example, which may allow the smoking substitute device to be charged by any USB-compatible device capable of delivering power to the smoking substitute device 10 via a suitable USB cable (in this case the USB-compatible device would be acting as the charging station 6). Alternatively, the charging station could be a docking station specifically configured to dock with the smoking substitute device 10 and charge the smoking substitute device 10 via the charging port on the smoking substitute device 10.

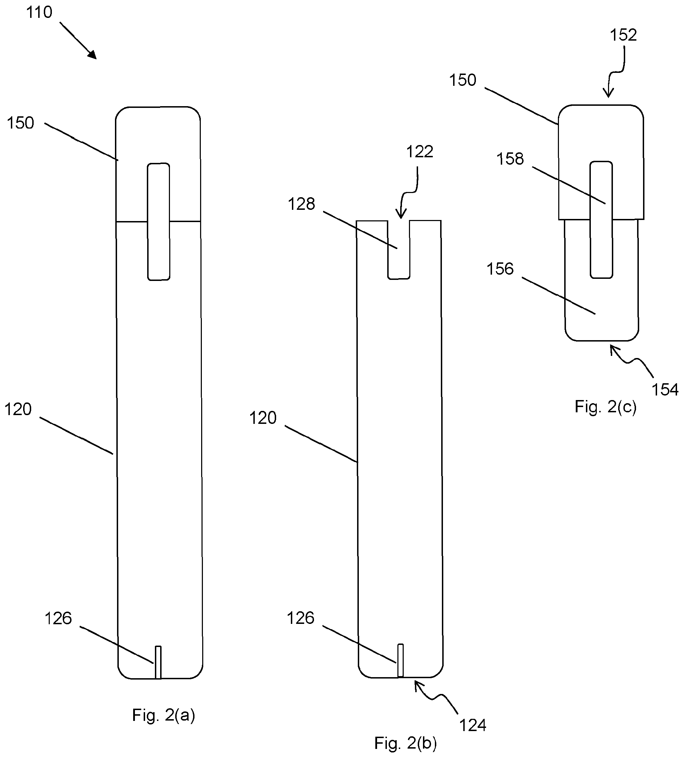

Fig. 2(a) shows an example smoking substitute device 110 for use as the smoking substitute device 10 in the system 1 of Fig. 1.

In this example, the smoking substitute device 110 includes a main body 120 and a consumable 150. The consumable 150 may alternatively be referred to as a "pod".

In this example, the smoking substitute device 110 is a closed system vaping device, wherein the consumable 150 includes a sealed tank 156 and is intended for one-use only.

Fig. 2(a) shows the smoking substitute device 110 with the main body 120 physically coupled to the consumable 150.

Fig. 2(b) shows the main body 120 of the smoking substitute device 110 without the consumable 150.

Fig. 2(c) shows the consumable 150 of the smoking substitute device 110 without the main body 120.

The main body 120 and the consumable 150 are configured to be physically coupled together, in this example by pushing the consumable 150 into an aperture in a top end 122 of the main body 120, e.g. with the consumable 150 being retained in the aperture via an interference fit. In other examples, the main body 120 and the consumable could be physically coupled together by screwing one onto the other, through a bayonet fitting, or through a snap engagement mechanism, for example. An optional light 126, e.g. an LED located behind a small translucent cover, is located a bottom end 124 of the main body 120. The light 126 may be configured to illuminate when the smoking substitute device 110 is activated.

The consumable 150 includes a mouthpiece (not shown) at a top end 152 of the consumable 150, as well as one or more air inlets (not shown in Fig. 2) so that air can be drawn into the smoking substitute device 110 when a user inhales through the mouthpiece. At a bottom end 154 of the consumable 150, there is located a tank 156 that contains e-liquid. The tank 156 may be a translucent body, for example.

The tank 156 preferably includes a window 158, so that the amount of e-liquid in the tank 156 can be visually assessed. The main body 120 includes a slot 128 so that the window 158 of the consumable 150 can be seen whilst the rest of the tank 156 is obscured from view when the consumable 150 is inserted into the aperture in the top end 122 of the main body 120.

In this present embodiment, the consumable 302 is a "single-use" consumable. That is, upon exhausting the e-liquid in the tank 156, the intention is that the user disposes of the whole consumable 150. In other embodiments, the e-liquid (i.e. aerosol former) may be the only part of the system that is truly "single-use". In such embodiments, the tank 156 may be refillable with e-liquid or the e-liquid may be stored in a non-consumable component of the system. For example, the e-liquid may be stored in a tank located in the device or stored in another component that is itself not single-use (e.g. a refillable tank).

The tank 156 may be referred to as a "clearomizer" if it includes a window 158, or a "cartomizer" if it does not.

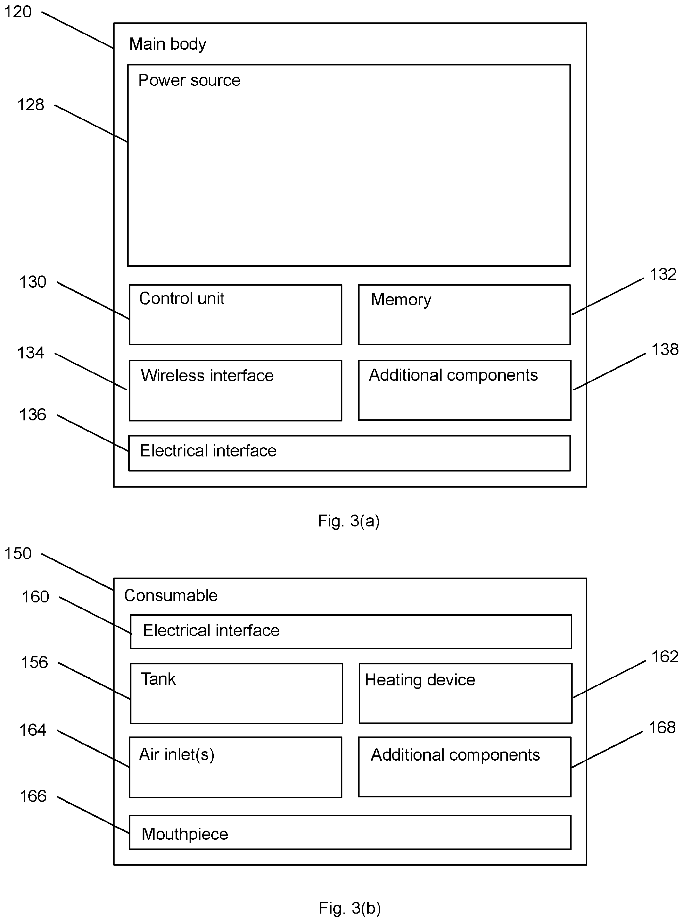

Fig. 3(a) is a schematic view of the main body 120 of the smoking substitute device 110.

Fig. 3(b) is a schematic view of the consumable 150 of the smoking substitute device 110.

As shown in Fig. 3(a), the main body 120 includes a power source 128, a control unit 130, a memory 132, a wireless interface 134, an electrical interface 136, and, optionally, one or more additional components 138.

The power source 128 is preferably a battery, more preferably a rechargeable battery.

The control unit 130 may include a microprocessor, for example.

The memory 132 preferably includes non-volatile memory.

The wireless interface establishes communication over a wireless communication channel between the smoking substitute device and communication terminal of the external device. The wireless interface may utilities any suitable wireless protocol. Suitably, the wireless interface may operate over a short range network. For example, it may comprise a wireless personal area network (WPAN), e.g. using Bluetooth™, ZigBee, a WiFi personal hotspot or the like. The smoking substitute device may pair with the portable communication terminal over the wireless communication channel. The portable communication terminal may be a master device and the smoking substitute device may be a slave device. As such, in exemplary embodiments, the wireless interface 134 is preferably configured to communicate wirelessly with the mobile device 2, e.g. via Bluetooth. To this end, the wireless interface 134 could include a Bluetooth™ antenna. Other wireless communication interfaces, e.g. WiFi, are also possible.

The electrical interface 136 of the main body 120 may include one or more electrical supply contacts. The electrical interface 136 may be located in, and preferably at the bottom of, the aperture in the top end 122 of the main body 120. When the main body 120 is physically coupled to the consumable 150, the electrical interface 136 may be configured to pass electrical power from the power source 128 to (e.g. a heating device of) the consumable 150 when the smoking substitute device 110 is activated, e.g. via the electrical interface 160 of the consumable 150 (discussed below). When the main body 120 is not physically coupled to the consumable 150, the electrical interface may be configured to receive power from the charging station 6.

The additional components 138 of the main body 120 may include the optional light 126 discussed above.

The additional components 138 of the main body 120 may, if the power source 128 is a rechargeable battery, include a charging port configured to receive power from the charging station 6. This may be located at the bottom end 124 of the main body 120. Alternatively, the electrical interface 136 discussed above is configured to act as a charging port configured to receive power from the charging station 6 such that a separate charging port is not required.

The additional components 138 of the main body 120 may, if the power source 128 is a rechargeable battery, include a battery charging control circuit, for controlling the charging of the rechargeable battery. However, a battery charging control circuit could equally be located in the charging station 6 (if present).

The additional components 138 of the main body 120 may include an airflow sensor for detecting airflow in the smoking substitute device 110, e.g. caused by a user inhaling through a mouthpiece 166 (discussed below) of the smoking substitute device 110. The smoking substitute device 110 may be configured to be activated when airflow is detected by the airflow sensor. This optional sensor could alternatively be included in the consumable 150 (though this is less preferred where the consumable 150 is intended to be disposed of after use, as in this example).

The additional components 138 of the main body 120 may include an actuator, e.g. a button. The smoking substitute device 110 may be configured to be activated when the actuator is actuated. This provides an alternative to the airflow sensor noted, as a mechanism for activating the smoking substitute device 110.

The additional components 138 of the main body 120 may include a motion sensor such as an accelerometer. The accelerometer may function as a motion sensor to receive inputs for controlling the device. An accelerometer is, as the skilled reader will know, an electromechanical device that measures acceleration forces, and provides a measure of "proper acceleration", which is the acceleration of a body or object, relative to free fall. The accelerometer comprised within the smoking substitute device, is configured to measure dynamic acceleration forces, and so can sense movement or vibrations. The accelerometer is configured to measure acceleration and its outputs may be used to determine position factors and/or orientation factors such as tilt, tilt angle, and incline, as well as being used to determine actions or events such as rotation, vibration and collision.

The accelerometer may be a piezoelectric accelerometer. However other types of accelerometer may be used in a smoking substitute device, such as a capacitance accelerometer. The accelerometer may comprise a three-axis model, to enable it to sense rotational tilt, as well as movement in a two-dimensional plane.

The accelerometer may be configured to detect movement and collisions, and to provide one or more voltage outputs to the control unit 130, as a result of what it has detected. The accelerometer can, for example, detect the action of the smoking substitute device being tapped against (i.e. relatively gently colliding with) a surface. When the user taps the device, the accelerometer transmits a corresponding voltage signal to the control unit 130. The control unit 130 can then control the memory 132 to store (at least temporarily) a measure of the voltage signal, along with an indicator of the time at which it was received. If the smoking substitute device is currently paired with, or bonded to, a mobile device, it may also submit a signal to the mobile device, via the wireless communication link that has been established between them, regarding the detection that the accelerometer has made. This can be very useful as the smoking substitute device may be preconfigured for a tap (or a plurality of taps) to form part of a sequence for the user to convey instructions to the device and/or to the connected mobile device or application.

The additional components 138 of the main body 120 may include a reading device configured to read information associated with the consumable from a machine readable data source included in (e.g. contained in the body of, or attached to) the consumable 150.

In some examples, the reading device (if present) may be configured to read information from the machine readable data source non-wirelessly, e.g. using an electrical connection between the main body 120 and consumable 150.

For example, the reading device (if present) may include a set of one or more electrical communication contacts configured to read information from the machine readable data source via an electrical connection established between the set of one or more electrical communication contacts and the machine readable data source. Conveniently, the set of one or more electrical communication contacts of the reading device may be configured to provide the electrical connection by engaging with a set of one or more electrical communication contacts of the consumable 150, when the main body 120 and the consumable 150 are physically coupled together.

In some examples, the reading device (if present) may be configured to read information from the machine readable data source wirelessly, e.g. via electromagnetic waves or optically. Thus, for example, the machine readable data source included in the consumable 150 could be an RFID tag (in which case the reading device included in the main body 120 may be an RFID reader) or a visual data source such as a barcode (in which case the reading device included in the main body may be an optical reader, e.g. a barcode scanner). Various wireless technologies and protocols may be employed to allow the reading device to wirelessly read information from a machine readable data source included in or attached to the consumable 150, e.g. NFC, Bluetooth, Wi-Fi, as would be appreciated by a skilled person.

The reading device (if present) may be configured to write information associated with the consumable to the machine readable data source (e.g. wirelessly or non-wirelessly, via one of the mechanisms discussed above) in addition to being configured to read information associated with the consumable from the machine readable data source. In this case, the reading device may be referred to as a reading/writing device.

As shown in Fig. 3(b), the consumable 150 includes the tank 156, an electrical interface 160, a heating device 162, one or more air inlets 164, a mouthpiece 166, and, optionally, one or more additional components 168.

The electrical interface 160 of the consumable 150 may include one or more electrical supply contacts. The electrical interface 136 of the main body 120 and an electrical interface 160 of the consumable 150 are preferably configured to contact each other and therefore electrically couple the main body 120 to the consumable 150 when the main body 120 is physically coupled to the consumable 150. In this way, electrical energy (e.g. in the form of an electrical current) is able to be supplied from the power source 128 in the main body 120 to the heating device 162 in the consumable 150.

The heating device 162 is preferably configured to heat e-liquid contained in the tank 156, e.g. using electrical energy supplied from the power source 128. In one example, the heating device 162 may include a heating filament and a wick, wherein a first portion of the wick extends into the tank 156 in order to draw e-liquid out from the tank 156, and wherein the heating filament coils around a second portion of the wick located outside the tank 156. In this example, the heating filament is configured to heat up e-liquid drawn out of the tank 156 by the wick to produce an aerosol vapour.

The one or more air inlets 164 are preferably configured to allow air to be drawn into the smoking substitute device 110, when a user inhales through the mouthpiece 166.

The additional components 168 of the consumable 150 may include a machine readable data source, which may e.g. be contained in the body of, or attached to the consumable 150. The machine readable data source may store information associated with the consumable. The information associated with the consumable may include information concerning the content of the consumable (e.g. e-liquid type, batch number) and/or a unique identifier, for example.

The machine readable data source may be rewritable, e.g. a rewritable RFID chip, or read only, e.g. a visual data source such as a barcode. As indicated above, the additional components 138 of the main body 120 may include a reading device configured to read information associated with the consumable from the machine readable data source.

For example, the electrical interface 160 of the consumable 150 may include a set of one or more electrical communication contacts, which may allow a reading device of the main body to read information from a machine readable data source of the consumable, e.g. as discussed previously.

In use, a user activates the smoking substitute device 110, e.g. through actuating an actuator included in the main body 120 or by inhaling through the mouthpiece 166 as described above. Upon activation, the control unit 130 may supply electrical energy from the power source 128 to the heating device 162 (via electrical interfaces 136, 166), which may cause the heating device 162 to heat e-liquid drawn from the tank 156 to produce a vapour which is inhaled by a user through the mouthpiece 166.

Of course, a skilled reader would readily appreciate that the smoking substitute device 110 shown in Figs. 2 and 3 shows just one example implementation of a smoking substitute device, and that other forms of smoking substitute device could be used as the smoking substitute device 10 of Fig. 1.

By way of example, a HNB smoking substitute device including a main body and a consumable could be used as the smoking substitute device 10 of Fig. 1, instead of the smoking substitute device 110. One such HNB smoking substitute device is the IQOS™ smoking substitute device discussed above.

As another example, an open system vaping device which includes a main body, a refillable tank, and a mouthpiece could be used as the smoking substitute device 10 of Fig. 1, instead of the smoking substitute device 110. One such open system vaping device is the blu PRO™ e-cigarette discussed above.

As another example, an entirely disposable (one use) smoking substitute device could be used as the smoking substitute device 10 of Fig. 1, instead of the smoking substitute device 110.

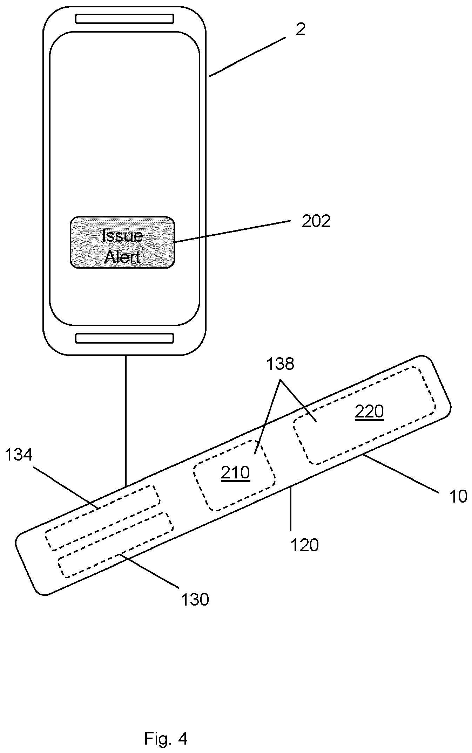

Referring to Figure 4, an exemplary embodiment is shown wherein the system comprises an external device 2 and a smoking substitute device 10. The devices 2, 10 can be as explained above, but specific features relating to the automatic deactivation of an alert are herein further described.

The external device is shown as suitably being a smart telephone operating an application. However, it will be appreciated that other devices such as tablet computers, smart watches or the like may be equally applicable. The external device 2 runs the application that can be used as a user interface between the user and the smoking substitute device. In order to establish communication between the external device and smoking substitute device, the smoking substitute device includes a communications interface and the external device is connected thereto. It is envisaged the connection will be a short distance wireless connection so that the user can be within distance of the alert in order to subsequently use the alert to guide them to the location of the smoking substitute device. Therefore, upon initial set up of the system, it will be appreciated that the application may be used to pair the external device with the smoking substitute device. For instance, the application may complete a Bluetooth pairing operation or another operation to establish the external device as a trusted device. With communication between the devices established, the external device can communicate with the smoking substitute device via the application and the smoking substitute device can communicate with the application by being controlled to transmit data via the communication interface. Thus the user can manipulate the external device to send the alert command wirelessly to the smoking substitute device. As shown in Figure 4 an input button 202 is suitably provide as part of the application's graphic user interface to enable a user to initiate the alert. Thus the smoking substitute device receives an alert command and the control unit recognises the receipt of the alert command as the first event trigger and activates the alert device.

The smoking substitute device's body 1 houses the wireless interface 134, the control unit 130 and additional components 138. In the exemplary embodiments, the additional components are a motion sensor 210 and an alert device 220.

In one exemplary embodiment, the motion sensor 210 is an accelerometer as herein described. It will be appreciated that the motion sensor is therefore able to sense and convert movement of the smoking substitute device into signals. Here, electronic signals for instance form the motion sensor are analysed and used by the control unit to determine whether the smoking substitute device has been moved to replicate a predetermined movement. If the control unit determines the motion sensor has detected a movement replicating a predetermined movement that corresponds to a second event trigger, the control unit 130 can deactivate the alert device 220.

In one exemplary embodiment, the alert device 220 issues a visual alert when activated. Here, it will be appreciated that the alert device may be a light emitter such as a light 126 as herein described.

In an additional or alternative exemplary embodiment, the alert device 220 issues an audible alert when activated. Here the alert device 220 may comprise a speaker or a haptic generator. Speakers are known in the art and the control unit 130 may control the speaker to issue an audible alert to assist the user locating the smoking substitute device when nearby. For instance the speaker could emit a periodic sound such as a buzz or beep or other noise until deactivated. Alternatively, the speaker may be controlled to issue a substantially constant sound such as music, wherein the music may be recognisable and attributed to the smoking substitute device.

In an additional or alternative exemplary embodiment, the alert device 200 is a haptic generator that causes a vibration force on the smoking substitute device when activated. Typically, when vibrated, the smoking substitute device emanates an audible alert that a user may recognise to assist in locating the device. In addition, if the device is in the user's clothing, the vibrations may transmit a haptic sensory alert to the user. The haptic generator may be a haptic motor and a weight mounted eccentrically on a shaft of the electric motor. The rotation of the eccentric weight produces the vibratory force.

It will be appreciated that in the exemplary embodiments, the body 120 may house a power source 128 and the control unit controls the activation and deactivation of the alert device by controlling the supply of power to the alert device from a power source 128.

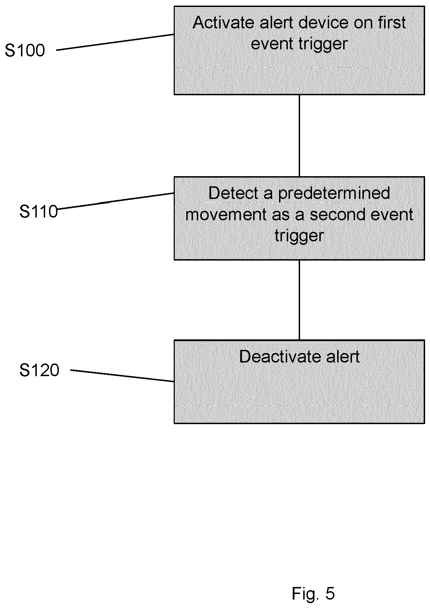

In an exemplary method a smoking substitute device is managed so that an alert device is automatically deactivated upon the motion sensor detecting that a predetermined movement of the smoking substitute device has been replicated. As shown in Figure 5, at step S100 the alert device 220 is activated upon the control unit 130 identifying a first event trigger. Suitably, the first event trigger is the receipt by the wireless interface of an alert command. Here, in an exemplary method of managing a system, the method includes the precursor step of a user inputting an alert command to cause the external device to transmit an alert command to the smoking substitute device connected thereto via a wireless communications link.

At step S110, the control unit is configured to detect a predetermined movement of the smoking substitute device has been replicated by monitoring and / or analysing the output of a motion sensor. Thus the step S110 may comprise using a motion sensor to detect a second event trigger, wherein the second event trigger is detection of a predetermined movement being replicated by the smoking substitute device.

Consequently the method comprises at step S130 deactivating the alert subsequent to identifying the second event trigger.

According to exemplary embodiments, the method of managing the system may comprise the optional step of verifying that a smoking substitute device is connected via a wireless communication link. If there is a wireless connection link, the method may comprise subsequently transmitting the alert command to the smoking substitute device. Alternatively, if the wireless link is not connected, the method may comprise issuing an error command. The error command may be a message through the application's graphic user interface or otherwise. Alternatively, the verification may occur before an alert function is active and the alert function is not activated to allow the alert command to be transmitted unless the wireless link is verified as being established.

According to another exemplary embodiment, after deactivating the alert, the method may comprise the step of transmitting a found command from the smoking substitute device back to the external device.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

## Claims

1. A smoking substitute device comprising:
a wireless communication interface for wirelessly communicating with an external device;
an alert device for issuing an alert;
a motion sensor for sensing motion of the smoking substitute device and
a control unit for controlling the alert device to be activated between a first event trigger and a second event trigger; wherein
the control unit is configured to determine the first event trigger as the wireless communication interface receiving an issue alert command from the external device; and
the control unit is configured to determine the second event trigger as the motion sensor detecting movement of the smoking substitute device corresponding to a predetermined movement.

2. The smoking substitute device of Claim 1, wherein the motion sensor is an accelerometer.

3. The smoking substitute device of Claim 1, wherein the alert device activates continuously between the first event trigger and the second event trigger.

4. The smoking substitute device of Claim 1, wherein the alert device activates periodically between the first event trigger and the second event trigger.

5. The smoking substitute device of Claim 1, wherein the alert device is a speaker.

6. The smoking substitute device of Claim 1, wherein the alert device is a light emitter.

7. The smoking substitute device of Claim 1, wherein the alert device is a haptic vibrator.

8. The smoking substitute device of Claim 1, wherein the predetermined movement replicates a lifting or picking up of the smoking substitute device.

9. The smoking substitute device of Claim 1, wherein the wireless communication interface establishes a short distance wireless link.

10. The smoking substitute device of Claim 1, wherein the control unit is configured to send a found command to the external device upon detection of the second trigger event.

11. A system comprising the smoking substitute device of Claim 1 and an external device, wherein the external device is configured to establish a wireless communication link with the smoking substitute device and to transmit an alert command to the smoking substitute device.

12. The system of Claim 11 wherein the external device is configured to verify whether the wireless communication link is established and if a link is not established to issue an error message.

13. A method of managing a smoking substitute device comprising the steps of;
activating an alert unit to issue an alert upon a control unit identifying a first event trigger;
detecting a predetermined movement of the smoking substitute device as a second event trigger; and
deactivating the alert unit automatically upon detection of the second event trigger.

14. The method of Claim 13, wherein the method includes the step of a user lifting the smoking substitute device subsequent to the step of activating an alert, wherein the control unit is configured to recognise the lifting motion as the second event trigger.

15. A computer implemented method for controlling a smoking substitute device to execute the method of claim 13 or a computer-readable medium containing computer-readable instructions which, when executed by a processor, cause the processor to perform the method of claim 13.
